# EUROPÄISCHE PATENTANMELDUNG

(11) **EP 1 813 587 A1**
(43) Veröffentlichungstag der Anmeldung: **01.08.2007**
(21) Anmeldenummer: 07000854.5
(22) Anmeldetag: 17.01.2007
(51) Int. Cl.: C07C 29/16, C07C 31/27, C07C 45/50, C07C 47/347

(54) **3(4),7(8)-Dihydroxymethyl-bicyclo[4.3.0] nonan und ein Verfahren zu seiner Herstellung**

(30) Priorität: 31.01.2006 DE 10604318
(71) Anmelder: OXEA Deutschland GmbH, 46147 Oberhausen (DE)
(72) Erfinder: Springer, Helmut, 46539 Dinslaken (DE); Bavaj, Paolo, Dr., 60320 Frankfurt (DE)

(57) **Zusammenfassung**

Die vorliegende Erfindung betrifft die chemische Verbindung 3(4),7(8)-Dihydroxymethyl-bicyclo[4.3.0]nonan sowie ein Verfahren zu ihrer Herstellung, wobei man Bicyclo[4.3.0]nona-3,7-dien in homogener organischer Phase in Gegenwart von Organophosphorverbindungen in komplexer Bindung enthaltenden Übergangsmetallverbindungen der Gruppe VIII des Periodensystems der Elemente und überschüssiger Organophosphorverbindung bei Temperaturen von 70 bis 160 °C und Drücken von 5 bis 35 MPa mit Synthesegas umsetzt und das so erhaltene 3(4),7(8)-Bisformyl-bicyclo[4.3.0]nonan hydriert.

## Beschreibung

Die vorliegende Erfindung betrifft die chemische Verbindung 3(4),7(8)-Dihydroxymethyl-bicyclo[4.3.0]nonan und ein Verfahren zu seiner Herstellung aus Bicyclo[4.3.0]nona-3,7-dien.

Kondensierte, alicyclische, ungesättigte Kohlenwasserstoffe mit isolierten Doppelbindungen in den Ringen sind wertvolle Ausgangsprodukte, die sich zu anwendungstechnisch wichtigen Verbindungen umsetzen lassen. Das ringförmig aufgebaute und kondensierte Kohlenwasserstoffgerüst verleiht dabei besondere Eigenschaften. Ein wichtiges Beispiel für diese Verbindungsklasse ist das durch Dimerisierung von Cyclopentadien leicht zugängliche und auch großtechnisch hergestellte Dicyclopentadien (DCP), das sich zu anwendungstechnisch wichtigen Verbindungen umsetzen lässt, denen das Tricyclodecan-Gerüst besondere Eigenschaften verleiht. Die vom DCPabgeleiteten Verbindungen mit Tricyclodecan-Struktur werden in der Literatur häufig auch als TCD-Derivate bezeichnet (Chemiker-Zeitung, 98, 1974, Seiten 70 bis 76).

Insbesondere die Hydroformylierung von DCP liefert interessante TCD-Aldehyde, wie 3(4),8(9)-Bisformyl-tricyclo[5.2.1.0^{2.6}]decan, auch als TCD-Dialdehyd bezeichnet, das zu wichtigen Zwischenprodukten weiterverarbeitet wird.

Wegen ihrer thermischen Labilität, die bei der destillativen Aufarbeitung zu Verlusten führt, wird TCD-Dialdehyd zumeist nicht rein isoliert, sondern als Rohprodukt der Hydroformylierungsreaktion weiterverarbeitet. So führt die Hydrierung von TCD-Dialdehyd zu TCD-Alkohol DM {3(4),8(9)-Dihydroxymethyl-tricyclo[5.2.1.0^{2.6}]decan}, das als wertvolles Zwischenprodukt für die chemische Industrie eine große wirtschaftliche Bedeutung besitzt. Der zweiwertige Alkohol ist in vielfältiger Weise für unterschiedliche Anwendungen von hohem technischen Interesse: Arylsäureester bzw. Methacrylsäureester von OH-gruppenhaltigen, tricyclischen Decanolen (DE 2 200 021 A), als Bestandteil von bei Sauerstoffausschluss erhärtenden Acrylsäureesterklebstoffen, (Meth)acrylsäureester von Ethergruppen enthaltenden tricyclischen Decanolen (EP 23 686 A2), zur Herstellung von Kleb- und Dichtungsstoffen, Ester und Polyester der Tricyclodecanreihe (DE 934 889 C), die als Weichmacher und hochwertige Esterschmierstoffe geeignet sind, Riechstoffkompositionen (DE 2 307 627 A1) und säuresterilisationsfeste Polyesterlacke (DE 3 134 640 C1) im Metall-Lackierungsbereich.

Die Herstellung von Aldehyden durch katalytische Anlagerung von Kohlenmonoxid und Wasserstoff an olefinische Doppelbindungen ist bekannt. Während diese Umsetzung früher nahezu ausschließlich mit Kobalt als Katalysator durchgeführt wurde, arbeiten moderne Verfahren mit metallischem Rhodium oder mit Rhodiumverbindungen als Katalysatoren, die allein oder mit komplexbildenden Liganden, z.B. organischen Phosphinen oder Estern der phosphorigen Säure, eingesetzt werden. Unter den Reaktionsbedingungen als Katalysator wirksam sind nach übereinstimmender Auffassung der Fachwelt Hydridocarbonylverbindungen des Rhodiums, die sich durch die allgemeine Formel H[Rh(CO)₄₋ₓLₓ] wiedergeben lassen, wobei L einen Liganden bezeichnet und x gleich 0 oder eine ganze Zahl von 1 bis 3 ist.

Einen Sonderfall stellt die Hydroformylierung von Dienen dar. Während bei der Hydroformylierung konjugierter Diene unter den üblichen Bedingungen der Oxo-Synthese fast ausschließlich Monoaldehyde erhalten werden, lassen sich aus Dicyclopentadien (DCP) mit seinen isolierten Doppelbindungen neben den Mono- auch die Disubstitutionsprodukte gewinnen. Aufgrund der großen Bedeutung der Hydroformylierungsprodukte des DCP finden sich auch in der technischen Literatur zahlreiche Arbeiten, die sich sowohl mit der Hydroformylierungsreaktion von DCP als auch mit der nachfolgenden Aufarbeitung des Rohproduktes befassen. So behandeln DE 38 22 038 A1 und GB 1 170 226 die Hydroformylierung von DCP in Gegenwart von Rhodium in einem organischen Lösungsmittel bei erhöhtem Druck und erhöhter Temperatur. Eine zusammenfassende Darstellung über die Hydroformylierung von Dicyclopentadien findet sich in der Chemiker-Zeitung 98, 1974, 70-76, wobei ebenfalls auf die thermische Labilität der TCD-Aldehyde hingewiesen wird, die bei der destillativen Aufarbeitung des rohen Hydroformylierungsgemisches zu hohen Produktverlusten führt. Daher werden TCD-Dialdehyde zumeist nicht rein isoliert, sondern in ihren Gemischen mit den Nebenprodukten der Oxo-Synthese weiterverarbeitet. Es finden sich im Stand der Technik, z.B. in EP-1 065 194 A1 oder US 5,138,101 A aber auch Hinweise auf extraktive Aufarbeitungsprozesse ohne thermische Belastung. Dabei wird das organische Rohgemisch mit einem polaren organischen Lösungsmittel, beispielsweise mit einem mehrwertigen Alkohol oder mit einem Methanol/Wasser-Gemisch extrahiert, wobei die TCD-Dialdehyde in die polare, alkoholische Phase übergehen und der Hydroformylierungskatalysator in der Kohlenwasserstoffphase verbleibt.

Aufgrund der vielseitigen Anwendungsmöglichkeiten besitzt TCD-Alkohol-DM ein hohes wirtschaftliches Interesse und in der Patentliteratur finden sich auch zahlreiche Hinweise auf Verfahren zu seiner Herstellung.

Das US-Patent US 4 647 708 A beschreibt die Hydroformylierung von Dicyclopentadien mit Rh als Katalysator in Gegenwart von lonenaustauschern (Dowex® MWA-1) in Toluol / THF als Lösungsmitteln. Die Umsetzung erfolgt bei 120°C und 27,5 MPa CO/H₂ (im Verhältnis 1 : 2) in zwei getrennten kontinuierlich betriebenen Autoklaven. Anhand der offenbarten Versuchsergebnisse kann man ersehen, dass die Ausbeute an TCD-Alkohol DM in dem 30 tägigen Versuchslauf von 85 % auf 65 % abfällt. Das Reaktionssystem ist somit für eine technische Anwendung nicht geeignet.

Im US-Patent US 4 262 147 A wird der Einsatz von bimetallischen Rh/Co-Clustern auf Harzen wie Amberlite® IRA-68 beschrieben. Unter den angewandten Bedingungen (110°C, 11 MPa, 8 Stunden) wird eine Selektivität von 68 % an TCD-Alkohol DM in dieser einstufigen Synthese erhalten.

Aufgrund der großen wirtschaftlichen Bedeutung, die Diole auf Basis von kondensierten, alicyclischen Kohlenwasserstoffen besitzen, besteht daher der Bedarf nach der Bereitstellung weiterer, preisgünstig verfügbarer Diole in hoher Reinheit, die ein ringförmig aufgebautes Kohlenwasserstoffgerüst mit kondensierten Ringen aufweisen.

Die vorliegende Erfindung betrifft daher die chemische Verbindung 3(4),7(8)-Dihydroxymethyl-bicyclo[4.3.0]nonan.

Ebenfalls besteht die Erfindung in einem Verfahren zur Herstellung von 3(4),7(8)-Dihydroxymethyl-bicyclo[4.3.0]nonan durch Hydroformylierung von Bicyclo[4.3.0]nona-3,7-dien mit nachfolgender Hydrierung. Es ist dadurch gekennzeichnet, dass man Bicyclo[4.3.0]nona-3,7-dien in homogener organischer Phase in Gegenwart von Organophosphorverbindungen in komplexer Bindung enthaltenden Übergangsmetallverbindungen der Gruppe VIII des Periodensystems der Elemente und überschüssiger Organophosphorverbindung bei Temperaturen von 70 bis 160°C und Drücken von 5 bis 35 MPa mit Synthesegas umsetzt und das so erhaltene 3(4),7(8)-Bisformylbicyclo[4.3.0]nonan anschließend zum 3(4),7(8)-Dihydroxymethylbicyclo[4.3.0]nonan hydriert.

Die erfindungsgemäße Verbindung leitet sich von Bicyclo[4.3.0]nona-3,7-dien ab, das technisch durch Diels-Alder-Reaktion von Butadien mit Cyclopentadien hergestellt wird und das daher in preisgünstigen Mengen zur Verfügung steht.

Die Zählung der in dem ungesättigten, bicyclischen Kohlenwasserstoff gebundenen Kohlenstoffatome erfolgt nach folgender Reihenfolge: 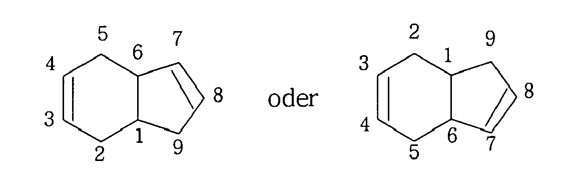 wobei beide Strukturformeln identisch sind.

Bei der erfindungsgemäßen Verbindung 3(4),7(8)-Dihydroxymethylbicyclo[4.3.0]nonan handelt es sich um ein Gemisch aus verschiedenen Isomeren des Dihydroxymethyl-bicyclo[4.3.0]nonans, bei denen die Hydroxymethylgruppe im Sechsring einmal an der 3- oder an der 4-Position und die Hydroxymethylgruppe im Fünfring einmal an der 7- oder an der 8-Position gebunden sein kann.

In Analogie zu der bei den TCD-Derivaten gemäß Chemiker-Zeitung, 98, 1974, Seiten 70 bis 76 üblichen Schreibweise kann die erfindungsgemäße Verbindung 3(4),7(8)-Dihydroxymethyl-bicyclo[4.3.0]nonan formelmäßig wie folgt beschrieben werden: 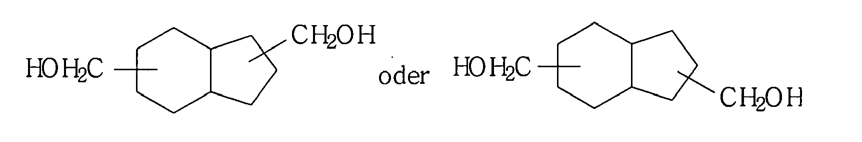 wobei beide Strukturformeln identisch sind.

Das Ausgangsprodukt Bicyclo[4.3.0]nona-3,7-dien kann als solches oder in Lösung der Hydroformylierung zugeführt werden. Geeignete Lösungsmittel sind solche, in denen Ausgangsmaterial, Reaktionsprodukt und Katalysator löslich sind und die sich unter den Reaktionsbedingungen inert verhalten, beispielsweise wasserunlösliche Ketone, Dialkylether, aliphatische Nitrile, aromatische Kohlenwasserstoffe wie Benzol, Toluol, die isomeren Xylole oder Mesitylen und gesättigte cycloaliphatische Kohlenwasserstoffe wie Cyclopentan oder Cylcohexan oder gesättigte aliphatische Kohlenwasserstoffe, wie n-Hexan, n-Heptan oder n-Octan. Der Anteil des Lösungsmittels im Reaktionsmedium kann über einen weiten Bereich variiert werden und beträgt üblicherweise zwischen 10 und 80 Gew.-%, vorzugsweise 20 bis 50 Gew.-%, bezogen auf das Reaktionsgemisch.

Die Hydroformylierungsstufe führt man in einem homogenen Reaktionssystem durch. Der Begriff homogenes Reaktionssystem steht für eine im wesentlichen aus Lösungsmittel, falls in der Reaktionsstufe zugesetzt, Katalysator, überschüssiger Organophosphorverbindung, unumgesetzter Ausgangsverbindung und Hydroformylierungsprodukt zusammengesetzte homogene Lösung.

Als Katalysatoren verwendet man Übergangsmetallverbindungen der Gruppe VIII des Periodensystems der Elemente, die Organophosphorverbindungen in komplexer Bindung enthalten. Vorzugsweise setzt man Komplexverbindungen des Kobalts, Rhodiums, Iridiums, Nickels, Eisens, Platins, Palladiums oder Rutheniums und insbesondere des Kobalts, Rhodiums und Iridiums ein. Besonders bevorzugt ist die Verwendung von Rhodium-Komplexverbindungen, die organische Phosphor(III)-Verbindungen als Liganden enthalten. Derartige Komplexverbindungen und ihre Herstellung sind bekannt (z.B. aus US 3 527 809 A, US 4 148 830 A, US 4 247 486 A, US 4 283 562 A). Sie können als einheitliche Komplexverbindungen oder auch als Gemisch unterschiedlicher Komplexverbindungen eingesetzt werden. Die Rhodium-Konzentration im Reaktionsmedium erstreckt sich über einen Bereich von 5 bis 1000 Gew.-ppm und beträgt vorzugsweise 10 bis 700 Gew.-ppm. Insbesondere wendet man Rhodium in Konzentrationen von 20 bis 500 Gew.-ppm, jeweils bezogen auf das homogene Reaktionsgemisch an. Die Hydroformylierung wird in Gegenwart eines Katalysatorsystems aus Rhodium-Organophosphor-Komplexverbindung und freiem, d.h. überschüssigen Organophosphor-Liganden durchgeführt, der mit Rhodium keine Komplexverbindung mehr eingeht. Der freie Organophosphor-Ligand kann der gleiche sein wie in der Rhodium-Komplexverbindung, es können aber auch von diesem verschiedene Liganden eingesetzt werden. Der freie Ligand kann eine einheitliche Verbindung sein oder aus einem Gemisch verschiedener Organophosphorverbindungen bestehen. Beispiele für Rhodium-Organophosphor-Komplexverbindungen, die als Katalysatoren Anwendung finden können, sind in US 3 527 809 A beschrieben. Zu den bevorzugten Liganden in den Rhodium-Komplexkatalysatoren zählen z. B. Triarylphosphine wie Triphenylphosphin, Trialkylphosphine wie Tri-(n-octyl)-phosphin, Trilaurylphosphin, Tri-(cyclohexyl)-phosphin, Alkylarylphosphine, Alkylphosphite, Arylphosphite, Alkyldiphosphite und Aryldiphosphite. So können Rhodium-Komplexverbindungen, die Arylphosphite der allgemeinen Formel P(OR¹)(OR²)(OR³) komplexgebunden enthalten, wobei wenigstens eine der Gruppen R¹, R² oder R³ für einen in ortho-Position substituierten Phenylring steht, ebenfalls eingesetzt werden. Als geeignete Komplexliganden haben sich Tris(2-tertiärbutylphenyl)phosphit oder Tris(2-tertiär-butyl-4-methylphenyl)phosphit erwiesen. Die Rhodium katalysierte Hydroformylierung von Olefinen mit Phosphit modifizierten Komplexverbindungen ist aus EP 0 054 986 A1 bekannt. Wegen seiner leichten Zugänglichkeit wird Triphenylphosphin besonders häufig angewandt.

Üblicherweise beträgt in der homogenen Reaktionsmischung das molare Verhältnis von Rhodium zu Phosphor 1 : 5 bis 1 : 200, jedoch kann der molare Anteil des Phosphors in Form organischer Phosphorverbindungen auch höher sein. Vorzugsweise setzt man Rhodium und organisch gebundenen Phosphor in molaren Verhältnissen von 1 : 10 bis 1 : 100 ein.

Verwendet man in der Hydroformylierungsstufe ein anderes Übergangsmetall der Gruppe VIII des Periodensystems der Elemente als Rhodium, so liegt die Konzentration an Übergangsmetall und das molare Verhältnis von Übergangsmetall zu Phosphor in den Bereichen, die man auch bei Rhodium wählt. Die jeweiligen optimalen Werte lassen sich in Abhängigkeit von dem jeweils eingesetzten Übergangsmetall durch einfache Routineversuche ermitteln.

Die Bedingungen, unter denen die Hydroformylierung abläuft, können innerhalb weiter Grenzen variieren und den individuellen Gegebenheiten angepasst werden. Sie hängen u.a. vom Einsatzmaterial, vom ausgewählten Katalysatorsystem und vom angestrebten Umsetzungsgrad ab. Üblicherweise führt man die Hydroformylierung von Bicyclo[4.3.0]nona-3,7-dien bei Temperaturen von 70 bis 160°C durch. Bevorzugt hält man Temperaturen von 80 bis 150°C und insbesondere von 90 bis 140°C ein. Der Gesamtdruck erstreckt sich über einen Bereich von 5 bis 35 MPa, vorzugsweise 10 bis 30 MPa und insbesondere 20 bis 30 MPa. Das molare Verhältnis von Wasserstoff zu Kohlenmonoxid bewegt sich üblicherweise zwischen 1:10 und 10:1, Mischungen, die Wasserstoff und Kohlenmonoxid im molaren Verhältnis 3:1 bis 1:3, insbesondere etwa 1:1, enthalten, sind besonders geeignet.

Man bildet den Katalysator üblicherweise aus den Komponenten Übergangsmetall oder Übergangsmetallverbindung, Organophosphorverbindung und Synthesegas unter den Bedingungen der Hydroformylierungsreaktion im Reaktionsgemisch. Es ist aber auch möglich, den Katalysator zunächst zu präformieren und ihn anschließend der eigentlichen Hydroformylierungsstufe zuzuführen. Die Bedingungen der Präformierung entsprechen dabei im allgemeinen den Hydroformylierungsbedingungen.

Zur Herstellung des Hydroformylierungskatalysators gelangt das Übergangsmetall der Gruppe VIII des Periodensystems der Elemente, insbesondere Rhodium, entweder in metallischer Form oder als Verbindung zum Einsatz. In metallischer Form verwendet man das Übergangsmetall entweder als feinverteilte Partikel oder in dünner Schicht auf einem Träger, wie Aktivkohle, Calciumcarbonat, Aluminiumsilikat, Tonerde niedergeschlagen. Als Übergangsmetallverbindungen eignen sich Salze aliphatischer Mono- und Polycarbonsäuren, wie Übergangsmetall-2-Ethylhexanoate, -Acetate, -Oxalate, -Propionate oder -Malonate. Weiterhin können Salze anorganischer Wasserstoff- und Sauerstoffsäuren, wie Nitrate oder Sulfate, die verschiedenen Übergangsmetalloxide oder auch Übergangsmetallcarbonylverbindungen, wie Rh₃(CO)₁₂, Rh₆(CO)₁₆, Co₂(CO)₈, Co₄(CO)₁₆, Fe(CO)₅, Fe₂(CO)₉, Ir₂(CO)₈, Ir₄(CO)₁₂ oder Übergangsmetall-Komplexverbindungen, z.B. Cyclopentadienylrhodiumverbindungen, Rhodiumacetylacetonat, Cyclopentadienylkobalt-Cyclooctadien-1,5, Fe(CO)₃-Cyclooctadien-1,5, [RhCl(Cyclooctadien-1,5]₂ oder PtCl₂(Cyclooctadien-1,5) eingesetzt werden. Übergangsmetallhalogenverbindungen kommen wegen ihres korrosiven Verhaltens der Halogenidionen weniger in Betracht.

Bevorzugt werden Übergangsmetalloxide und insbesondere Übergangsmetallacetate und -2-ethylhexanoate. Als besonders geeignet haben sich Rhodiumoxid, Rhodiumacetat, Rhodium-2-ethylhexanoat, Kobaltoxid, Kobaltacetat und Kobalt-2-ethylhexanoat erwiesen.

Die Hydroformylierungsstufe kann sowohl absatzweise als auch kontinuierlich durchgeführt werden. Nach dem erfindungsgemäßen Verfahren wird das Ausgangsolefin Bicyclo[4.3.0]nona-3,7-dien nahezu vollständig umgesetzt und es wird ein rohes Hydroformylierungsprodukt mit einem hohen Gehalt an dem gewünschten Bisformylprodukt erhalten, der im allgemeinen über 75 Gew.-%, bezogen auf das rohe Hydroformylierungsprodukt, liegt.

Das Umsetzungsprodukt der Hydroformylierungsstufe wird ohne weitere Reinigung und ohne Katalysatorabtrennung der Hydrierstufe zugeführt.

Die Hydrierung des rohen 3(4),7(8)-Bisformyl-bicyclo[4.3.0]nonans zum 3(4),7(8)-Dihydroxymethyl-bicyclo[4.3.0]nonan erfolgt unter allgemein üblichen Reaktionsbedingungen in Gegenwart konventioneller Hydrierkatalysatoren. Im allgemeinen beträgt die Hydriertemperatur 70 bis 170°C und der angewandte Druck 1 bis 30 MPa. Als Hydrierkatalysatoren sind besonders Nickelkatalysatoren geeignet.

Das katalytisch aktive Metall kann auf einem Träger aufgebracht werden, im allgemeinen in einer Menge von etwa 5 bis etwa 70 Gew.-%, vorzugsweise etwa 10 bis etwa 65 Gew.-% und insbesondere etwa 20 bis etwa 60 Gew.-% jeweils bezogen auf das Gesamtgewicht des Katalysators. Als Katalysatorträger eignen sich alle herkömmlichen Trägermaterialien, z.B. Aluminiumoxid, Aluminiumoxidhydrate in ihren verschiedenen Erscheinungsformen, Siliciumdioxid, Polykieselsäuren (Kieselgele) einschließlich Kieselgur, Kieselxerogele, Magnesiumoxid, Zinkoxid, Zirconiumoxid und Aktivkohle. Neben den Hauptkomponenten Nickel und Trägermaterial können die Katalysatoren noch Zusatzstoffe in untergeordneten Mengen enthalten, die z.B. der Verbesserung ihrer Hydrieraktivität und/oder ihrer Standzeit und/oder ihrer Selektivität dienen. Derartige Zusatzstoffe sind bekannt, zu ihnen gehören z.B. die Oxide des Natriums, Kaliums, Magnesiums, Calciums, Bariums, Zinks, Aluminiums, Zirconiums und Chroms. Sie werden dem Katalysator im allgemeinen in einem Anteil von insgesamt 0,1 bis 50 Gew.-Teile bezogen auf 100 Gew.-Teile Nickel zugesetzt.

Aber auch trägerfreie Katalysatoren, wie Raney-Nickel oder Raney-Kobalt können in dem Hydrierprozess verwendet werden.

Die Hydrierstufe wird diskontinuierlich oder kontinuierlich in flüssiger Phase mit suspendierten Katalysatoren oder in flüssiger oder gasförmiger Phase mit fest angeordneten Katalysatoren durchgeführt; die kontinuierliche Arbeitsweise wird bevorzugt.

Bei diskontinuierlicher Verfahrensführung verwendet man, bezogen auf 3(4), 7(8)-Bisformyl-bicyclo[4.3.0]nonan, 1 bis 10, vorzugsweise 2 bis 6 Gew.-% Nickel in Form der vorstehend beschriebenen Katalysatoren. Bei kontinuierlicher Arbeitsweise setzt man je Liter Katalysator und Stunde etwa 0,05 bis etwa 5,0 kg des 3(4),7(8) Bisformyl-bicyclo[4.3.0]nonans ein, bevorzugt werden etwa 0,1 bis 2,0 kg 3(4),7(8)-Bisformyl-bicyclo[4.3.0]nonan je Liter Katalysator und Stunde.

Die Hydrierung erfolgt vorzugsweise mit reinem Wasserstoff. Es können jedoch auch Gemische verwendet werden, die freien Wasserstoff und daneben unter den Hydrierbedingungen inerte Bestandteile enthalten. In jedem Fall ist dafür Sorge zu tragen, dass das Hydriergas frei von Katalysatorgiften wie Schwefelverbindungen oder Kohlenmonoxid in schädlichen Mengen ist.

Rohes 3(4),7(8)-Bisformyl-bicyclo[4.3.0]nonan kann als solches oder zusammen mit einem Lösungs- oder Verdünnungsmittel eingesetzt werden, wobei die zuletzt genannte Variante aufgrund der hohen Viskosität des gebildeten Diols bevorzugt wird. Setzt man ein Lösungs- oder Verdünnungsmittel hinzu, ist die Auswahl der Lösungs- oder Verdünnungsmittel, die reine Substanzen aber auch Substanzgemische sein können, nicht kritisch sofern sichergestellt ist, dass sie mit dem Einsatzstoff und dem Reaktionsprodukt eine homogene Lösung bilden. Beispiele für geeignete Lösungs- oder Verdünnungsmittel sind lineare oder cyclische Ether wie Tetrahydrofuran oder Dioxan, sowie aliphatische Alkohole, beispielsweise Methanol, Ethanol, Butanol und Isobutanol. Die Menge des eingesetzten Lösungs- oder Verdünnungsmittels kann entsprechend den apparativen und verfahrenstechnischen Gegebenheiten frei gewählt werden, im allgemeinen setzt man Lösungen ein, die 10 bis 75 Gew.-% 3(4),7(8)-Bisformyl-bicyclo[4.3.0]nonan enthalten.

Die Gewinnung des reinen 3(4),7(8)-Dihydroxymethyl-bicyclo[4.3.0]nonans erfolgt nach konventionellen Destillationsverfahren. Das cyclische Diol wird als Kopfprodukt abgezogen. Restmengen des in der Hydroformylierungsstufe eingesetzten Übergangsmetalls fallen im Destillationsrückstand an und werden nach bekannten Verfahren zurückgewonnen.

Das Reaktionsprodukt der Hydroformylierung von Bicyclo[4.3.0]nona-3,7-dien kann aber auch zunächst nach konventionellen Verfahren destilliert werden und als gereinigtes Produkt hydriert werden. Überraschenderweise kann 3(4),7(8)-Bisformyl-bicyclo[4.3.0]nonan mit hoher destillativer Ausbringung in reiner Form erhalten werden. Dies ist um so mehr überraschend, weil der Stand der Technik auf die thermische Labilität von Dialdehyden mit kondensierten, alicyclischen Ringstrukturen hinweist. Übergangsmetall, vorzugsweise Rhodium, und zugesetzte Organophosphorverbindung fallen im Destillationsrückstand an und werden nach bekannten Methoden zurückgewonnen. Die anschließende Hydrierung des gereinigten 3(4),7(8)-Bisformylbicyclo[4.3.0]nonans erfolgt wie bei der Umsetzung des rohen Hydroformylierungsprodukts.

Das erfindungsgemäße Verfahren gestattet einen einfachen und kostengünstigen Zugang zu 3(4),7(8)-Dihydroxymethyl-bicyclo[4.3.0]nonan in hoher Ausbeute und in hoher Reinheit. Das nach dem erfindungsgemäßen Verfahren hergestellte Diol lässt sich in ausgezeichneter Weise für unterschiedliche Anwendungen einsetzen, beispielsweise als Bestandteil in Polyurethanen, Polyestern oder Acrylsäureestern sowie zur Herstellung von Folgeprodukten, die als Weichmacher oder Schmierstoffe verwendet werden.

Im Folgenden wird das erfindungsgemäße Verfahren näher erläutert, es ist jedoch nicht auf die beschriebenen Ausführungsformen beschränkt.

### Beispiele

### Herstellung von 3(4),7(8)-Dihydroxymethyl-bicyclo[4.3.0]nonan

### 1. Herstellung von 3(4),7(8)-Bisformyl-bicyclo[4.3.0]nonan

In einem Stahlautoklaven mit Magnetrührer wurden 1000 g Bicyclo[4.3.0]nona-3,7-dien, technische Qualität, und 1000 g Toluol vorgelegt. Nach Zusatz von 12,75 g Triphenylphosphin sowie 50 mg Rh, in Form einer toluolischen Lösung von Rh-2-ethylhexanoat mit einem Gehalt von 7062 mg Rh/kg, wurde das Gemisch auf 130°C erwärmt und unter einem Druck von 26 MPa mit Synthesegas behandelt. Nach einer Reaktionszeit von 8 Stunden wurde die Hydroformylierungsreaktion beendet.

Die organische Phase wurde gaschromatographisch untersucht.

| GC-Analyse (Flächenprozent ohne Toluol) | |
|---|---|
| Vorlaufkomponenten | 0,2 |
| Bicyclo[4.3.0]nona-3,7-dien-Bereich | 0,1 |
| Komponenten | 4,6 |
| 3(4),7(8)-Bisformyl-bicyclo[4.3.0]nonan | 89,1 |
| Triphenylphosphin/Triphenylphosphinoxid | 1,2 |
| Hochsieder | 4,8 |

### 2. Herstellung von 3(4),7(8)-Dihydroxymethyl-bicyclo[4.3.0]nonan

Das nach der Hydroformylierung erhaltene rohe 3(4),7(8)-Bisformylbicyclo[4.3.0]nonan wurde durch Destillation an einem Dünnschichtverdampfer weitgehend vom Toluol befreit (Manteltemperatur 140 °C, Druck 100 hPa). Es fiel ein Rückstand an, der nach gaschromatographischer Analyse neben 6,7 % Toluol und 83,8 % 3(4),7(8)-Bisformyl-bicyclo[4.3.0]nonan noch 9,5 % sonstige Komponenten enthielt. Nachfolgend wurden 700 g des Rückstands, verdünnt mit 300 g Isobutanol, und 42 g Ni 52/35-Katalysator der Firma Johnson Matthey Plc - in einem 3-1 Autoklav vorgelegt. Das Reaktionsgemisch wurde auf 130°C erwärmt und bei einem Druck von 10,0 MPa und einer Reaktionszeit von 8 Stunden umgesetzt. Nach Reaktionsende wurde abgekühlt, entspannt und vom Katalysator abfiltriert. Das so erhaltene Reaktionsprodukt wurde gaschromatographisch untersucht.

| GC-Analyse (in Flächenprozent) | |
|---|---|
| Vorlaufkomponenten | 1,3 % |
| Isobutanol/Toluol / Methylcyclohexan | 29,2 % |
| 3(4),7(8)-Dihydroxymethyl-bicyclo[4.3.0]nonan | 62,8 % |
| Sonstige | 6,7 % |

Zur Aufarbeitung wurde das rohe Hydrierprodukt an einer Claisenbrücke destilliert. Beim Einsatz von 825,3 g erhielt man 459,3 g Hauptfraktion in einem Siedebereich von 178-179°C bei einem Druck von 1 hPa mit folgender Zusammensetzung:

| GC-Analyse (in Flächenprozent) | |
|---|---|
| Vorlaufkomponenten | 0,1 % |
| 3(4),7(8)-Dihydroxymethyl-bicyclo[4.3.0]nonan | 97,3 % |
| Sonstige | 2,6 % |

Die Gesamtausbeute an 3(4),7(8)-Dihydroxymethyl-bicyclo[4.3.0]nonan beträgt über alle Stufen 79,6 % d.Th., bezogen auf eingesetztes Bicyclo[4.3.0]nona-3,7-dien.

### Charakterisierung:

| Elementaranalyse | | | |
|---|---|---|---|
| C₁₁H₂₀O₂ (184,3) | Ber. C 71,7 %, | H 10,9 %, | O 17,3 % |
| | Gef. C 70,6 %, | H 10,5 %, | O 16,7 % |

NMR-Daten
¹H-NMR (500 MHz, DMSO-d₆, ppm): 0,60-2,20 (m, 14 H, CH und CH₂), 3,18-3,43 (m, 4H, CH₂O), 4,32 (s, 2 H, OH)
¹³C-NMR (125 MHz, DMSO-d₆, ppm): 23,46-48,82 (CH und CH₂), 62,39-66,80 (CH₂OH)
IR-Daten (Diamant-ATR-IR Spektroskopie)
ν (cm⁻¹) 3306 (m, br), 2911 (s), 2855 (s), 1445 (w), 1030 (s)

| | |
|---|---|
| Dichte bei 60°C | 1,051 g/cm³ |
| Brechzahl n_{D} bei 60°C | 1,5000 |

Das erfindungsgemäße Verfahren eröffnet einen eleganten Herstellungsweg für 3(4),7(8)-Dihydroxymethyl-bicyclo[4.3.0]nonan in hohen Ausbeuten. Die neue Verbindung 3(4),7(8)-Dihydroxymethyl-bicyclo[4.3.0]nonan weist eine alicyclische Ringstruktur mit kondensierten Ringen auf, die sich hervorragend als Bestandteil für Polyurethane, Polyester oder Acrylsäureester eignet. Ebenfalls kann sie zur Herstellung von Folgeprodukten verwendet werden, die als Weichmacher und Schmierstoffe zum Einsatz kommen.

## Patentansprüche

1. Verfahren zur Herstellung von 3(4),7(8)-Dihydroxymethylbicyclo[4.3.0]nonan durch Hydroformylierung von Bicyclo[4.3.0]nona-3,7-dien mit nachfolgender Hydrierung, **dadurch gekennzeichnet, dass** man Bicyclo[4.3.0]nona-3,7-dien in homogener organischer Phase in Gegenwart von Organophosphorverbindungen in komplexer Bindung enthaltenden Übergangsmetallverbindungen der Gruppe VIII des Periodensystems der Elemente und überschüssiger Organophosphorverbindung bei Temperaturen von 70 bis 160 °C und Drücken von 5 bis 35 MPa mit Synthesegas umsetzt und das so erhaltene 3(4),7(8)-Bisformyl-bicyclo[4.3.0]nonan anschließend zum 3(4),7(8)-Dihydroxymethylbicyclo[4.3.0]nonan hydriert.

2. Verfahren gemäß Anspruch 1, **dadurch gekennzeichnet, dass** man als Organophosphorverbindungen organische Phosphor(III)-Verbindungen ausgewählt aus der Gruppe von Triarylphosphinen, Trialkylphosphinen, Alkylarylphosphinen, Alkylphosphiten, Arylphosphiten, Alkyldiphosphiten oder Aryldiphosphiten einsetzt.

3. Verfahren nach Anspruch 2, **dadurch gekennzeichnet, dass** man als Triarylphosphin Triphenylphosphin und als Arylphosphit Tris(2-tertiärbutylphenyl)phosphit oder Tris(2-tertiär-butyl-4-methylphenyl)phosphit einsetzt.

4. Verfahren gemäß einem oder mehreren der Ansprüche 1 bis 3, **dadurch gekennzeichnet, dass** als Übergangsmetallverbindungen der Gruppe VIII des Periodensystems der Elemente Verbindungen von Rhodium, Kobalt, Iridium, Nickel, Palladium, Platin, Eisen oder Ruthenium verwendet werden.

5. Verfahren gemäß einem oder mehreren der Ansprüche 1 bis 4, **dadurch gekennzeichnet, dass** als Übergangsmetallverbindungen der Gruppe VIII des Periodensystems der Elemente Verbindungen von Rhodium eingesetzt werden.

6. Verfahren gemäß einem oder mehreren der Ansprüche 1 bis 5, **dadurch gekennzeichnet, dass** man Rhodium in einer Konzentration von 5 bis 1000 Gew.-ppm, bezogen auf das homogene Reaktionsgemisch einsetzt.

7. Verfahren gemäß Anspruch 6, **dadurch gekennzeichnet, dass** man Rhodium in einer Konzentration von 10 bis 700 Gew.-ppm, vorzugsweise von 20 bis 500 Gew.-ppm, bezogen auf das homogene Reaktionsgemisch, einsetzt.

8. Verfahren gemäß einem oder mehreren der Ansprüche 1 bis 7, **dadurch gekennzeichnet, dass** das molare Verhältnis von Rhodium zu Phosphor 1 : 5 bis 1 : 200 beträgt.

9. Verfahren gemäß Anspruch 8, **dadurch gekennzeichnet, dass** das molare Verhältnis von Rhodium zu Phosphor 1 : 10 bis 1: 100 beträgt.

10. Verfahren gemäß einem oder mehreren der Ansprüche 1 bis 9, **dadurch gekennzeichnet, dass** man bei der Hydroformylierung bei Temperaturen von 80 bis 150°C, vorzugsweise von 90 bis 140°C, und bei Drücken von 10 bis 30 MPa, vorzugsweise von 20 bis 30 MPa, arbeitet.

11. Verfahren gemäß einem oder mehreren der Ansprüche 1 bis 10, **dadurch gekennzeichnet, dass** man die Hydrierung in Gegenwart von Nickelkatalysatoren bei Temperaturen von 70 bis 170°C und bei Drücken von 1 bis 30 MPa durchführt.

12. Die chemische Verbindung 3(4),7(8)-Dihydroxymethylbicyclo[4.3.0]nonan.

## Geänderte Patentansprüche

### Geänderte Patentansprüche gemäss Regel 86(2) EPÜ.

**1.** Verfahren zur Herstellung von 3(4),7(8)-Dihydroxymethylbicyclo[4.3.0]nonan durch Hydroformylierung von Bicyclo[4.3.0]nona-3,7-dien mit nachfolgender Hydrierung, **dadurch gekennzeichnet, dass** man Bicyclo[4.3.0]nona-3,7-dien in homogener organischer Phase in Gegenwart von Organophosphorverbindungen in komplexer Bindung enthaltenden Übergangsmetallverbindungen der Gruppe VIII des Periodensystems der Elemente und überschüssiger Organophosphorverbindung bei Temperaturen von 70 bis 160 °C und Drücken von 5 bis 35 MPa mit Synthesegas umsetzt und das so erhaltene 3(4),7(8)-Bisformyl-bicyclo[4.3.0]nonan anschließend zum 3(4),7(8)-Dihydroxymethylbicyclo[4.3.0]nonan hydriert.

**2.** Verfahren gemäß Anspruch 1, **dadurch gekennzeichnet, dass** man als Organophosphorverbindungen organische Phosphor(III)-Verbindungen ausgewählt aus der Gruppe von Triarylphosphinen, Trialkylphosphinen, Alkylarylphosphinen, Alkylphosphiten, Arylphosphiten, Alkyldiphosphiten oder Aryldiphosphiten einsetzt.

**3.** Verfahren nach Anspruch 2, **dadurch gekennzeichnet, dass** man als Triarylphosphin Triphenylphosphin und als Arylphosphit Tris(2-tertiärbutylphenyl)phosphit oder Tris(2-tertiär-butyl-4-methylphenyl)phosphit einsetzt.

**4.** Verfahren gemäß einem oder mehreren der Ansprüche 1 bis 3, **dadurch gekennzeichnet, dass** als Übergangsmetallverbindungen der Gruppe VIII des Periodensystems der Elemente Verbindungen von Rhodium, Kobalt, Iridium, Nickel, Palladium, Platin, Eisen oder Ruthenium verwendet werden.

**5.** Verfahren gemäß einem oder mehreren der Ansprüche 1 bis 4, **dadurch gekennzeichnet, dass** als Übergangsmetallverbindungen der Gruppe VIII des Periodensystems der Elemente Verbindungen von Rhodium eingesetzt werden.

**6.** Verfahren gemäß einem oder mehreren der Ansprüche 1 bis 5, **dadurch gekennzeichnet, dass** man Rhodium in einer Konzentration von 5 bis 1000 Gew.-ppm, bezogen auf das homogene Reaktionsgemisch einsetzt.

**7.** Verfahren gemäß Anspruch 6, **dadurch gekennzeichnet, dass** man Rhodium in einer Konzentration von 10 bis 700 Gew.-ppm, vorzugsweise von 20 bis 500 Gew.-ppm, bezogen auf das homogene Reaktionsgemisch, einsetzt.

**8.** Verfahren gemäß einem oder mehreren der Ansprüche 1 bis 7, **dadurch gekennzeichnet, dass** das molare Verhältnis von Rhodium zu Phosphor 1 : 5 bis 1 : 200 beträgt.

**9.** Verfahren gemäß Anspruch 8, **dadurch gekennzeichnet, dass** das molare Verhältnis von Rhodium zu Phosphor 1 : 10 bis 1: 100 beträgt.

**10.** Verfahren gemäß einem oder mehreren der Ansprüche 1 bis 9, **dadurch gekennzeichnet, dass** man bei der Hydroformylierung bei Temperaturen von 80 bis 150°C, vorzugsweise von 90 bis 140°C, und bei Drücken von 10 bis 30 MPa, vorzugsweise von 20 bis 30 MPa, arbeitet.

**11.** Verfahren gemäß einem oder mehreren der Ansprüche 1 bis 10, **dadurch gekennzeichnet, dass** man die Hydrierung in Gegenwart von Nickelkatalysatoren bei Temperaturen von 70 bis 170°C und bei Drücken von 1 bis 30 MPa durchführt.
